# EUROPEAN PATENT SPECIFICATION

(11) **EP 2 766 067 B1**
(45) Date of publication and mention of the grant of the patent: **22.04.2020**
(21) Application number: 12840823.4
(22) Date of filing: 09.10.2012
(51) Int. Cl.: A61M 5/00, A61M 37/00

(54) **INTEGRATED MICRONEEDLE ARRAY DELIVERY SYSTEM**
INTEGRIERTES MIKRONADELFELDFREISETZUNGSSYSTEM
SYSTÈME INTÉGRÉ D'ADMINISTRATION D'UN RÉSEAU DE MICRO-AIGUILLES

(30) Priority: 12.10.2011 US 201161546357 P
(43) Date of publication of application: 20.08.2014
(73) Proprietor: 3M Innovative Properties Company, St. Paul, MN 55133-3427 (US)
(72) Inventor: COLBURN, David J., Saint Paul, Minnesota 55133-3427 (US); JOHNSON, Erik J., Oakdale, Minnesota 55128 (US); BRANDWEIN, David H., Saint Paul, Minnesota 55133-3427 (US); GYSBERS, Jerome E., Saint Paul, Minnesota 55133-3427 (US); YOUNG, Patrick J., Saint Paul, Minnesota 55133-3427 (US); CANTOR, Adam S., Saint Paul, Minnesota 55133-3427 (US)
(74) Representative: Vossius & Partner Patentanwälte Rechtsanwälte mbB
(86) International application number: PCT/US2012/059276
(87) International publication number: WO 2013/055641

(56) References cited:
- EP-A2- 2 566 568
- WO-A1-2004/009172
- WO-A2-2011/140240
- US-A1- 2002 087 182
- US-A1- 2004 087 893
- US-A1- 2008 009 811
- US-A1- 2008 114 298
- US-A1- 2008 114 298
- US-A1- 2009 198 189
- US-A1- 2009 198 189
- US-A1- 2010 222 743
- US-A1- 2011 172 645
- US-B2- 7 097 631

## Description

### Background

Only a limited number of molecules with demonstrated therapeutic value can be transported through the skin, even with the use of approved chemical enhancers. The main barrier to transport of molecules through the skin is the stratum corneum (the outermost layer of the skin).

Devices including arrays of relatively small structures, sometimes referred to as microneedles or micro-pins, have been disclosed for use in connection with the delivery of therapeutic agents and other substances through the skin and other surfaces. The devices are typically pressed against the skin in an effort to pierce the stratum corneum such that the therapeutic agents and other substances can pass through that layer and into the tissues below. Microneedles of these devices pierce the stratum corneum upon contact, making a plurality of microscopic slits which serve as passageways through which molecules of active components can be delivered into the body. In delivering an active component, the microneedle device can be provided with a reservoir for temporarily retaining an active component in liquid form prior to delivering the active component through the stratum corneum. In some constructions, the microneedles can be hollow to provide a liquid flow path directly from the reservoir and through the microneedles to enable delivery of the therapeutic substance through the skin. In alternate constructions, active component(s) may be coated on the microneedle array and delivered directly through the skin after the stratum corneum has been punctured.

Microneedle arrays can be used in conjunction with an applicator device capable of being used several times or single-use. The microneedle arrays are generally used once and then discarded.

Issues related to applying microneedles include the ability to effectively and consistently insert the needles to a desired depth in the skin, the ability to reliably hold the microneedles in proper contact with the skin during the period of administration, and the ability to apply consistent force for delivery.

US 2009/198189 A1 relates to a device for applying a microneedle array to a skin surface. The device comprises a base, defining a skin contacting plane, an array component having a skin facing side comprising a microneedle array, and at least one connecting member having a first portion affixed through a first hinge to the base and a second portion affixed to the array component. The connecting member has a first equilibrium position with the microneedle array in a recessed position within the device and a second equilibrium position with the microneedle array positioned so as to be able to contact a skin surface.

US 2008/114298 A1 relates to an application device for applying a microneedle device to a skin surface comprising a flexible sheet having a raised central area attached to the microneedle device and a supporting member at or near the periphery of the flexible sheet, wherein the flexible sheet is configured such that it will undergo a stepwise motion in the direction orthogonal to the major plane of the sheet.

### Summary

The present invention is defined by the claims.

The present disclosure provides a low-profile system for delivering a microneedle array. The delivery system includes a housing that may be secured to and temporarily worn on a patient's skin. A carrier assembly coupled to a microneedle array is received in the housing proximate an applicator device. The applicator device can include an actuator and a stored energy device. In those implementations, the actuator can be moved relative to the housing to trigger the stored energy device, which will contact at least a portion of the carrier assembly and drive the array toward the target surface. The stored energy device can be designed to store a predetermined amount of potential energy that is greater or substantially greater than the energy necessary to release said energy. In certain embodiments, the potential energy released is at least twice the energy necessary to "activate". In other implementations of the delivery system, the kinetic energy can be at least 20 times greater than the activation energy. As the stored energy device is potentially capable of releasing substantially more energy than is required to cause the release, the amount of normal force applied to the skin can be minimized while still generating sufficient application velocity. As an additional result, the user to user variability in force applied to the carrier assembly can be reduced. This reduced variability can result in more consistent and repeatable microneedle penetration.

Certain implementations of the delivery system can be activated without creating a significant skin dome and without substantially stretching the skin or otherwise disturbing the surface. Certain actuators can be configured within a device housing such that no or substantially no force is applied normal to the skin by the user during movement of the actuator. While a small activation force in a direction orthogonal to the skin may be necessary to activate a stored energy device, the forces generated normal to the skin surface can be essentially reacted against one another within the housing. Accordingly, the force actually applied to the skin in a direction normal to the plane of the skin can be zero or near zero. In addition, the forces generated in a direction parallel to the plane of the skin in such embodiments are at least substantially transferred to the stored energy device. Thus, the efficacy of the motion of the actuator can discourage the user from pushing the device in a direction normal to the skin during activation, further increasing the consistency of application.

In delivery system embodiments that include a stored energy device, the variability in force applied to the array may be reduced. In certain previous delivery systems, increasing the amount of energy transferred to the microneedle array meant an increase in the amount of applied energy by the user or the distance the microarray traveled before reaching the skin. A stored energy device is configured to store a certain amount of potential energy that can be released upon transfer of a predetermined amount of activation energy to a surface of the device. By placing a stored energy device between the user-applied force and the carrier assembly, the velocity at which the array impacts the skin may be more closely regulated. The above benefits can be realized in an applicator that is easy to handle, simple to use, low cost, and suitable for disposal or reuse.

The present disclosure provides an integrated system for delivering a microneedle array. In certain implementations, the system includes a housing having a cavity therein and an actuator coupled thereto. An array carrier assembly including a solid microneedle array is received in the cavity and a stored energy device is coupled to a portion of the interior of the housing and is in contact with at least a portion of the carrier assembly. The actuator is operable to supply an activation energy to the stored energy device in a direction relative to the major plane of the array. The actuator is operable to apply a force to the stored energy device in a direction orthogonal to the major plane of the array, the application of the force causing a transfer of activation energy to the stored energy device.

The stored energy device is capable of transferring an application energy that is greater than the activation energy. In some implementations, the ratio of application energy to activation energy is greater than 5. The stored energy device includes a bifurcating spring, that undergoes a stepwise motion in a direction ortogonal to a major plane of the spring. The stored energy device is capable of transferring an application energy that is greater than the activation energy. In certain embodiments, the stored energy device comprises an aperture, which receives the carrier assembly, the carrier assembly comprising an elongated arm, which is releasably coupled to the housing at a location remote from the array. In certain embodiments, the actuator is movable within the housing in a certain direction substantially parallel to the attachment surface.

The present disclosure also provides methods for delivering a microneedle array to a patient's skin surface. In some aspects a method comprises: providing an integrated applicator including a housing having a cavity therein, a microneedle array received in the cavity, a stored energy device proximate the array, and an actuator slidably coupled to the housing; placing the applicator proximate the skin surface; moving the actuator relative to the housing, wherein moving the actuator comprises applying a force to the actuator, wherein the activation energy is transferred to the stored energy device by movement of the actuator; and transferring energy to the array, thereby driving the array against the skin surface, wherein the energy transferred to the array comprises an application energy, and wherein the application energy is greater than the activation energy.

In another implementation, a method for delivering a microneedle array includes providing a housing having a cavity therein, an array device received in the cavity, and an actuator slidably coupled to the housing; moving the actuator relative to the housing; and transferring activation energy to the array, wherein the force experienced normal to the patient's skin is zero or near zero during transfer.

As used herein, "activation energy" refers to the minimum amount of energy required to release the potential energy stored within a stored energy device.

As used herein, "application energy" refers to the energy released upon activation of a stored energy device and applied to a microneedle carrier.

As used herein, "array" refers to the medical devices described herein that include one or more structures capable of piercing the stratum comcum to facilitate the transdermal delivery of therapeutic agents or the sampling of fluids through or to the skin. "Microstructure," "microneedle" or "microarray" refers to the specific microscopic structures associated with the array that are capable of piercing the stratum comeum to facilitate the transdermal delivery of therapeutic agents or the sampling of fluids through the skin.

As used herein, "carrier assembly" refers to at least a microneedle array and any structure used to couple the array to a housing. For example, the carrier assembly can refer to an array, a flexible membrane, and an adhesive layer. As another example, the carrier assembly refers to the array and the array carrier.

As used herein, "solid microneedle array" means an array comprised of microneedles of any size and shape that do not have an exposed bore therethough., in contrast to hollow microneedle arrays.

As used herein, "travel distance" refers to the distance traveled by an element of the delivery system upon actuation. For example, the travel distance for a stored energy device may be different than the travel distance for the array.

The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As recited herein, all numbers should be considered modified by the term "about".

As used herein, "a," "an," "the," "at least one," and "one or more" are used interchangeably. Thus, for example, a delivery apparatus comprising "a" stored energy device can be interpreted to comprise "one or more" stored energy devices.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exhaustive list.

### Brief Description of the Drawings

The invention will be further described with reference to the drawings, wherein corresponding reference characters indicate corresponding parts throughout the several views, and wherein:
Figure 1 is a perspective view of a microneedle delivery system according to one embodiment of the present disclosure.
Figure 2 is a perspective view of the microneedle delivery system of Figure 1.
Figure 3 is an exploded view of the microneedle delivery system of Figure 1.
Figure 4 is a cross-sectional view of the microneedle delivery system of Figure 1.
Figures 5A-C are cross-sectional views of the delivery system of the previous figures in operation.
Figure 6 is a perspective view of a bifurcating spring according to certain embodiments of the disclosure.
Figure 7 is a perspective view of a bifurcating spring according to another aspect of the disclosure.
Figure 8 is a perspective view of a microneedle delivery system according to another aspect of the disclosure (not claimed).
Figure 9 is a cross-sectional view of the delivery system of Figure 8.
Figure 10 is a perspective view of a microneedle delivery system according to another aspect of the disclosure (not claimed).
Figure 11 is a cross-sectional view of the delivery system of Figure 10.
Figures 12 is a perspective view of a perspective view of a microneedle delivery system according to yet another aspect of the disclosure.
Figure 13 is a cross-sectional view of the delivery system of Figure 12.
Figure 14 depicts the delivery system of Figure 12 in operation.

While the above-identified figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art.

### Detailed Description of Illustrative Embodiments

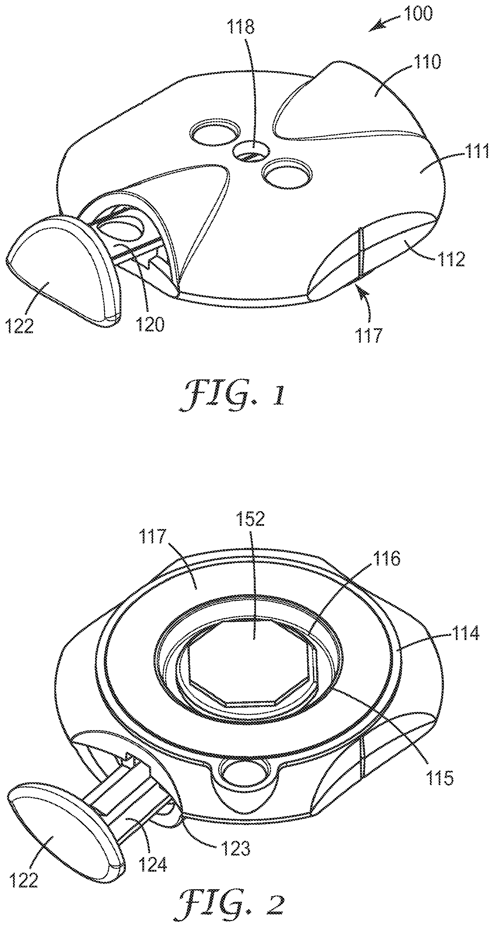

One embodiment of a delivery system is depicted in Figures 1-4. A delivery system 100 includes a device housing 110. The housing 110 can be self-contained and compactly constructed to provide a relatively low profile and small footprint for, among other factors, ease of use and patient comfort. In the embodiment illustrated Figures 1 and 2, the housing 110 may include lower housing portion 112 and mating upper housing portion 111. Alternatively (though not depicted), the delivery system may include a unitary housing. Upper and lower housing portions 111 and 112 may be coupled together by any suitable means including, but not limited to, snap-fit together or coupled by hinge, frictional interference fits, adhesive, welding, heat-staking, solvent bonding, mechanical fasteners, and the like. Housing 110 may be made of suitable lightweight materials compatible for ease of patient and practitioner handling. The materials used in housing 110 may include, but are not limited to, plastics, metals, composite materials, and combinations thereof. For example, the housing 110 can be made of thermoplastics such as polypropylene, polybutylene terephthalate, polystyrene, polyethylene, polythermide, polyethylene terephthalate, polystyrene, polyvinyl chloride, polymethylmethacrylate, acrylonitrile-butadiene styrene, polycarbonate, and blends thereof. Other possible materials include metals, such as aluminum, steel, and stainless steel. Further, upper housing portion 111 may include a window 118 that allows a user to easily visually observe the operation of the elements within the cavity 116. Additionally or alternatively, the upper housing portion 111 can include transparent material to allow a user to visually inspect the application of a microneedle array.

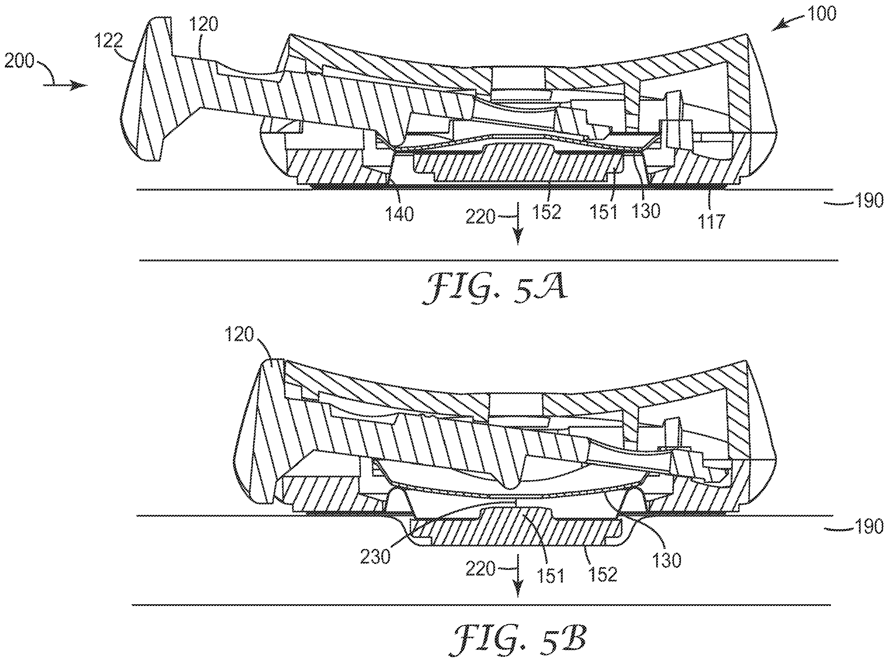

The housing 110 includes a cavity 116 that receives a carrier assembly 150. The carrier assembly 150 includes an array carrier 151 and a microneedle array 152 coupled to a surface thereof. The microneedle array comprises a major plane that is oriented generally parallel to a skin surface 190 (as depicted in Figure 5A) during use of the delivery system. Microneedle array 152 can include one or more needle or needle-like structures as well as other structures capable of piercing the stratum corneum. The microneedles are typically less than 900 microns, often less than 600 microns in height, and sometimes less than 300 microns in height. The microneedles are typically more than 20 microns in height, often more than 50 microns in height, and sometimes more than 125 microns in height. A stored energy device 130 is received proximate to or within the cavity 116 near a surface of the carrier assembly 150. The stored energy device can also be in direct contact with a portion of the carrier assembly 150. In other embodiments, the distance between the assembly 150 and the stored energy device 130 can vary, allowing the stored energy device to travel a certain distance before contacting the assembly 150.

The microneedles useful in the various embodiments of the invention may comprise any of a variety of configurations, including but not limited to those described in the following patents and patent applications. One embodiment for the microneedles comprises the structures disclosed in United States Patent No. 6,881,203. The disclosed microstructures in the aforementioned patent application are in the form of microneedles having tapered structures that include at least one channel formed in the outside surface of each microneedle. The microneedles may have bases that are elongated in one direction. The channels in microneedles with elongated bases may extend from one of the ends of the elongated bases towards the tips of the microneedles. The channels formed along the sides of the microneedles may optionally be terminated short of the tips of the microneedles. The microneedle arrays may also include conduit structures formed on the surface of the substrate on which the microneedle array is located. The channels in the microneedles may be in fluid communication with the conduit structures. Another embodiment for the microneedle devices comprises the structures disclosed in co-pending United States Patent Publication No.US2005/0261631 which describes microneedles having a truncated tapered shape and a controlled aspect ratio. Still another embodiment for the microneedles comprises the structures disclosed in United States Patent No. 6,091,975 (Daddona, et al.) which describes blade-like microprotrusions for piercing the skin. Still another embodiment for the microneedles comprises the structures disclosed in United States Patent No. 6,312,612 (Sherman, et al.) which describes tapered structures having a hollow central channel. Still another embodiment for the micro arrays comprises the structures disclosed in International Publication No. WO 00/74766 (Garstein, et al.) which describes hollow microneedles having at least one longitudinal blade at the top surface of tip of the microneedle.

The cavity 116 can be defined by cooperation of both the upper housing 111 and the lower housing 112, or may be solely contained in the lower housing 112. The minimum height of the cavity 116 is influenced by the desired travel distance of the microneedle array 152 before reaching the skin surface and the travel distance of the stored energy device. Accordingly, the height of cavity 116 in some embodiments may be no greater than 2 centimeters. In other embodiments, the height of the cavity 116 may be no greater than 1 centimeter, in other embodiments no greater than 8 millimeters, in yet other embodiments no greater than 5 millimeters. In certain embodiments, the height of the cavity is at least 1 millimeter in other embodiments at least 2 millimeters, in other embodiments at least 5 millimeters. Cavities less than 1 millimeter in height may not allow sufficient travel distance for the array to pierce the stratum corneum and/or may require application of greater force to the array than safe or desirable in a low-profile device.

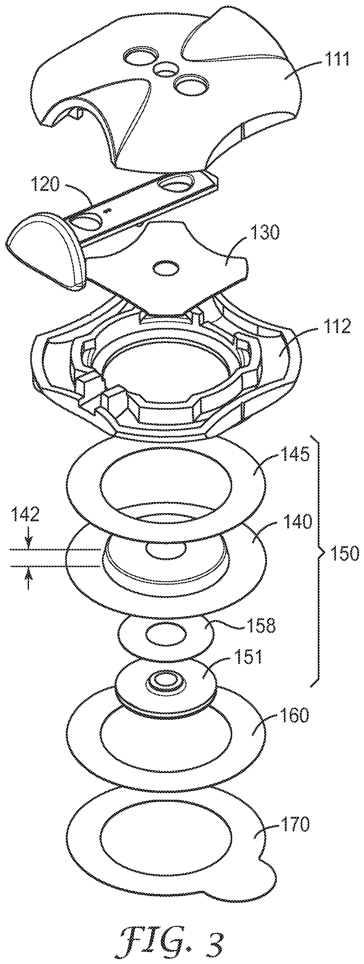

Lower housing portion 112 includes a base 114, which can be generally planar, and defines an opening 115 to the cavity 116. The base 114 includes an attachment surface 117, which at least partially envelopes the opening 115 and can also be generally planar. The attachment surface 117 can include an adhesive layer 160 for eventual attachment of the housing 110 to a patient's skin surface. Adhesive layer 160 can be a continuous coating, a patterned coating, or discrete portions of adhesive, or combinations thereof. In certain embodiments, a first major surface of the adhesive can be coupled to a release liner 170 (See Fig. 3) prior to use.

It may be desired that the height of the housing be designed for ease of handling and operation. Accordingly, the height of the housing 110 may be no greater than 3 centimeters. In other embodiments, the height of the housing may be no greater than 1 centimeter, in other embodiments no greater than 5 millimeters, in other embodiments no greater than 3 millimeters. In certain embodiments, the height of the housing is at least 1.5 millimeters, in other embodiments at least 2 millimeters, in other embodiments at least 5 millimeters. Housings less than 1.5 millimeters in height may not allow sufficient travel distance for the array to pierce the stratum corneum and may be too difficult to handle. On the other end of the spectrum, housings greater than 3 centimeters in height can be unwieldy and difficult to maintain adherence to skin.

Referring now to Figures 2 and 3, additional aspects of the delivery system 100 are further detailed. An applicator device for use in triggering the system can include a stored energy device 130 and an actuator 120. In certain embodiments, the stored energy device 130 can be secured to the interior of the housing by any suitable attachment means, including but not limited to adhesives, fasteners, interference-fits, and the like. In certain embodiments, peripheral portions of the stored energy device 130 may be contained between the upper housing 111 and lower housing 112, and rested on a groove or ridge portion proximate the cavity 116 without any additional attachment. The stored energy device 130 is actuatable to apply force in a direction generally orthogonal to the attachment surface 117 and major plane of the array 152. Suitable stored energy devices include, but are not limited to, deflected beams, coiled springs, leaf-like springs, domed springs, propellant canisters, and the like. In most embodiments, a portion of the stored energy device can travel unimpeded within the housing and/or cavity to deliver the application force to the carrier assembly 150.

The stored energy device 130 is actuatable for applying force (i.e., application energy) to carrier assembly 150, thereby accelerating at least a portion of the assembly to a velocity before impact sufficient to pierce the skin. It is desirable that a consistent, predetermined amount of force is necessary to actuate the stored energy device, thereby resulting in a consistent amount of force applied normal to the skin during actuation. The microneedle array 152 typically reaches a velocity before impact ranging from between about 2 and about 20 m/sec before the microneedle array impacts a patient's skin. More typically, the array strikes a patient's skin at a velocity before impact ranging from between about 4 and about 15 m/sec. In certain preferred embodiments, the velocity at impact is consistently over 10 m/sec. It can also be desirable to limit the velocity to prevent or reduce the stimulation of underlying nerve tissue on or after impact.

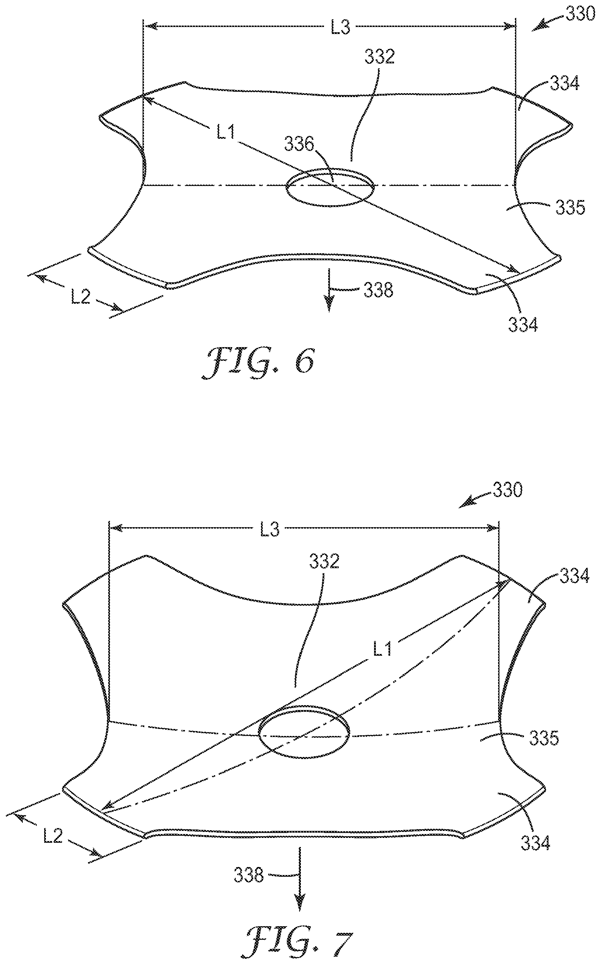

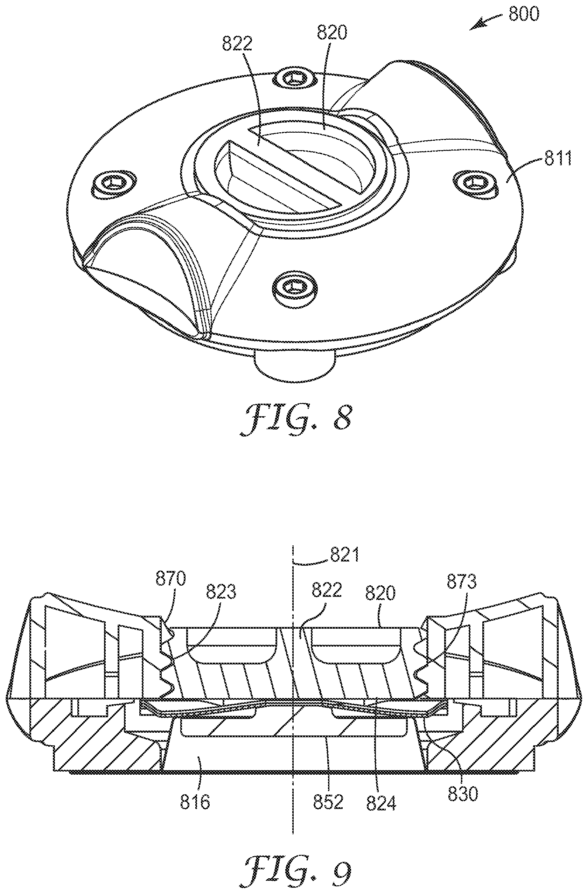

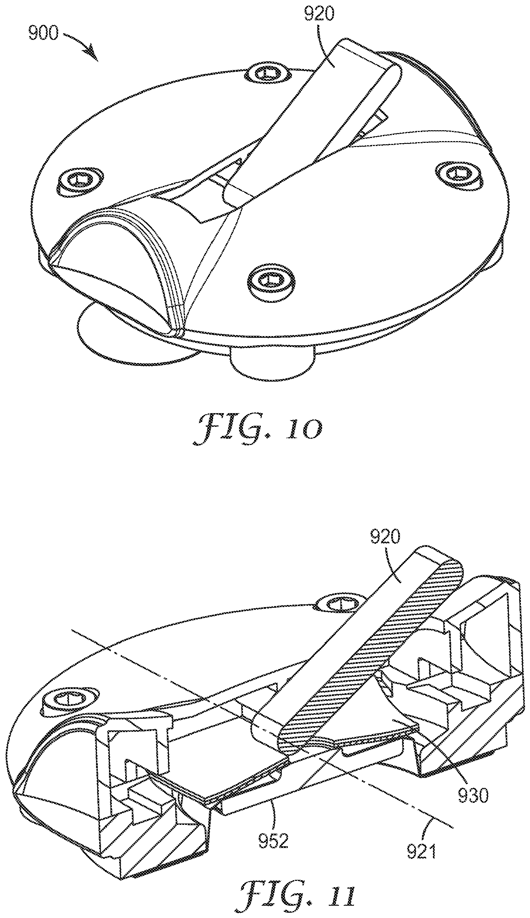

In certain embodiments, the stored energy device 130 is configured so that it will undergo a bifurcated (i.e., stepwise) motion in a direction generally orthogonal to the attachment surface and/or the major plane of a microneedle array. For example, the stored energy device in such embodiments can be a domed, bifurcating spring as depicted in Figures 6 and 7. The stored energy device can also include a plurality of bifurcating springs, as depicted in Figures 9 and 11. As used herein, a bifurcating spring is a spring that undergoes a shape change as a result of a predetermined force applied normal to a major plane of the spring. Using methods further described herein, it is possible to manufacture bifurcating springs that can store a greater or substantially greater amount of energy than can be comfortably applied to the delivery system by a user during use.

One embodiment of such a bifurcating spring is depicted in a loaded, stable configuration (i.e., a state prior to exterior application of energy) in Figure 6. In addition to the depicted springs, other suitable springs include Belleville washers and domed springs. The undulating spring 330 includes a generally circular central portion and one or more legs 334. In certain embodiments, the spring 330 can include an aperture 336 proximate the center. The legs 334 are at least partially defined by an arcuate section 335. The loaded spring 330 is designed to store a certain amount of potential energy. This potential energy is converted to a kinetic energy once a predetermined amount of activation energy is transferred to the first major surface 332 of the spring 330. This results in the spring bifurcating and reaching the second stable configuration as depicted in Figure 7. The release of kinetic energy occurs in a direction 338 generally orthogonal to the major plane of the spring, which results in the center of the spring traveling a certain distance between first and second stable states.

Due to the potential energy stored within, the bifurcating spring 330 may release energy greater than the energy necessary to cause bifurcation (i.e., the activation energy). In certain embodiments, the kinetic energy (i.e., application energy when transferred to a microneedle array or carrier assembly) is at least twice the activation energy. In certain embodiments, the application energy is 4 times greater than the activation energy, in other embodiments, at least 10 times, and in yet other embodiments at least 20 times greater than the activation energy. As the bifurcating spring is potentially capable of releasing substantially more energy than is required to cause bifurcation, the amount of normal force applied to the skin can be minimized while still generating sufficient application velocity. As an additional result, the user to user variability in force applied to the carrier assembly can be reduced. This reduced variability can result in more consistent and repeatable microneedle penetration.

A bifurcating spring according to the present disclosure may be created by, e.g., applying a predetermined force (i.e., load) to the center of an otherwise non-bifurcating domed spring, while supporting the periphery. Suitable non-bifurcating springs include but are not limited to stainless steel domed springs available from Snaptron Inc., Windsor, CO. The force applied to a spring surface is preferably sufficient to cause plastic deformation of at least a portion of the spring. In certain embodiments, a press or probe applies a displacement to a surface 332 of the spring at a constant rate. The press continues past the point of bifurcation and until the desired force has been applied after bifurcation. At this point, the probe stops moving and remains in place for a given amount of hold time. In the case of a "zero" hold time, the press begins to retract immediately after reaching the desired bend force. Without wishing to be bound by theory, both the load applied after bifurcation and the time over which the load is applied appear to be positively correlated with the energy necessary to activate the spring, and to a lesser degree, the potential energy released on bifurcation. After a certain time, however, an increase in hold time may not amount to an appreciable increase in stored energy. The energy applied after activation of the spring is further influenced by, *inter alia,* the material of the spring, the thickness of the material, the number of legs, dimensions of the arcuate sections between the legs, and the geometry (e.g., height, diameter) of the spring. By manipulating at least these variables, both the energy required to activate the bifurcating spring and the energy applied to a carrier assembly can be tailored to suit the needs of a desired microneedle delivery system.

The housing 110 further includes an actuator 120. The actuator 120 cooperates with the stored energy device 130 to form an applicator device. The actuator 120 includes a finger engageable portion 122 that is adapted to cover actuator opening 123 formed in the upper housing portion 111. In the embodiment depicted in Figures 1-5, the actuator further includes an extended arm portion 124 that extends from finger engageable portion 122 through opening 123 and into the cavity 116. The arm portion 124 can include a wedge 126 or other protrusion. The actuator 120 is movable within the housing at an angle relative to the attachment surface 117. Movement of the wedge 126 relative to the stored energy device 130 applies a force in a direction generally orthogonal to the major plane of the array 152.

In other embodiments, the actuator may not actually contact the stored energy device 130, but can remove an impediment to the release of kinetic energy. For example, the actuator may engage and deform a releasable retaining device that holds the stored energy device in a primed position. Deformation or displacement of the releasable retaining device may then allow the stored energy device to discharge the potential energy stored therein.

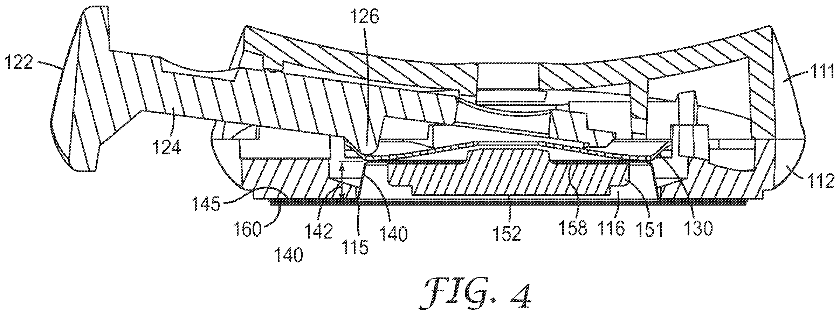

As depicted in Figures 3 and 4, carrier assembly 150 includes a solid microneedle array 152, an array carrier 151, and a flexible membrane 140. The microneedle array 152 can be attached to a first surface of array carrier 151 by any suitable attachment means. As shown in Figure 3, the microneedle array can also be formed or molded as an integral portion of the array carrier 151. In other embodiments, the attachment means is an adhesive, which may be in the form of a continuous coating, a patterned coating, or discrete portions of adhesive. In one aspect, the adhesive attachment is non-permanent, that is, after application of the microneedle array, the carrier may be removed from the skin surface though all or part of the array remains. Other suitable attachment means for connecting the microneedle device 152 and the array carrier 151 include snap-fit connections, hook and loop (e.g., Velcro™) attachments, magnetic attachment, heat bonding, welding, or any other suitable attachment method known to one of ordinary skill in the art.

The microneedle array 152 depicted in Figure 2 has an octagonal shape, but any of a number of shapes and sizes are suitable for use with application devices of the present invention. The array 152 and/or array carrier 151 may be disposable or reusable.

At least a portion of the array carrier 151 can be formed so as to be relatively rigid. Suitable materials include polymers, such as liquid crystal polymers, polypropylene, polybutylene terephthalate, polystyrene, polyethylene, polythermide, polyethylene terephthalate, polystyrene, polyvinyl chloride, polymethylmethacrylate, acrylonitrile-butadiene styrene, polycarbonate, and blends thereof. The rigidity can provide support for the microneedle array and may assist in the transfer of application energy. Other materials are also contemplated, including metals, ceramics, and other materials that will be apparent to those skilled in the art. In certain embodiments, the array carrier is comprised of the same material as the microneedles of the microneedle array.

In certain circumstances, it may be desirable for the array 152 and/or the array carrier 151 to include a unique identifier on an exposed surface. For example, a bar code can be printed on the array or array carrier using heat transfer, ink jet, laser inscribing, or other bar code printing methods. In other embodiments, the bar code can be printed on e.g., adhesive tape and adhered to a surface of the array or array carrier. Such as a bar code or other type of identification marking can be used to quickly access information regarding the array and/or array carrier. RFID tags, two-dimensional optically detectable codes, or the like, may also be used as unique identifiers. This unique identifier can improve the traceability of individual components of the delivery system.

The carrier assembly 150 can include a flexible membrane 140 coupled to a surface of the array carrier 151 opposite the microneedle array 152 via attachment mechanism 158. The flexible membrane 140 includes a chamber having a bellowed height 142. The bellowed height 142 is preferably such that the carrier assembly 150 is retained in contact with or in close proximity to a portion of the stored energy device 130 when received in the cavity 116. The flexible membrane 140 is preferably capable of retaining the bellowed height 142 prior to the application of force to the carrier assembly 150 via the stored energy device. For example, the membrane can be constructed of CoTran 9701 polyurethane film, available from 3M Company, St. Paul, MN. Other polymeric films capable of maintaining the bellowed height are also suitable for use as a membrane. In certain embodiments, the flexible membrane includes a material that is sterilizable and/or maintains a sterile barrier.

The flexible membrane 140 can be vented or non-vented. When the delivery system 100 is placed on the skin surface, a chamber of air may be formed between the skin surface, the adhesive 160, the membrane/array assembly, and potentially a portion of the lower housing 112. As used herein, a membrane is "vented" when it includes deliberate apertures or channels to allow the flow of fluid out of the chamber. In certain preferred embodiments, at least a portion of the membrane is non-vented, in that is does not include any deliberate means for fluid to flow out of the chamber. Non-vented membranes may surprisingly provide better depth of microneedle penetration in the skin, as well as more consistent penetration levels across the entire array. Furthermore, a non-vented membrane may allow for a carrier assembly to be provided pre-sterilized and pre-loaded with the agent intended for transdermal delivery.

The carrier assembly 150 may be secured within the cavity 116 via attachment of the flexible membrane 140. For example, a certain length of the flexible membrane 140 can be secured via attachment mechanism 145 to portions of the lower housing 112 proximate the opening 115. Additionally or alternatively, the chamber can be sized to create an interference fit with cavity 116, such that the carrier assembly 150 may simply be pressed into the housing.

The carrier assembly 150, or a portion thereof, can also be coupled or releasably coupled to the stored energy device 130. For example, the stored energy device 130 may include one or more apertures and array carrier 151 can include one or more elongated protrusions capable of being received in the apertures. Once so received, the protrusion may be staked to the stored energy device 130. In other embodiments, the carrier assembly 150 may be secured to the stored energy device 130 via adhesive or other attachment means described herein.

In embodiments wherein the carrier assembly is attached or releasably attached to a stored energy device comprising a spring, the motion of the microneedle array is coupled to that of the spring. After activation and transfer of application energy (i.e., the release of the stored potential energy in the spring and its subsequent contact with the some portion of the carrier assembly), both the spring and carrier assembly will move cooperatively towards the skin surface in the direction of the applied force. Impact of the assembly at the skin surface will also cause the skin to move in the direction of the applied force. At some point after activation, the spring reaches a point of maximum extension and will begin to travel away from the skin (i.e., recoil). The surface of the skin, however, may continue to move in the direction of the applied force. Since they are coupled to motion of the spring, the microneedles may stop penetrating or even pull out of the skin, leading to less and/or more inconsistent depth of penetration.

In one exemplary embodiment of the present disclosure, the stored energy device 130 is not attached or otherwise fixed to the carrier assembly 150. As such, following impact at the skin, the stored energy device 130 may freely recoil upwardly and vibrate without otherwise affecting the travel of the carrier assembly/microneedle array. Since the carrier assembly 150 is not attached to the stored energy device 130 after activation, it is free to continue moving forward with the motion of the skin; without regard to the recoil of the stored energy device 130. This independent motion may reduce the tendency for the microneedles to stop penetrating or to be pulled out of the skin. Increased depth and more consistent penetration of the microneedles can result in improved delivery across the stratum corneum.

The carrier assembly 150 can also be coupled to the housing 110 or cavity 116 without use of a flexible membrane 140 and without attachment to the stored energy device 130. For example, the array carrier can include protrusions that rest on an internal ridge or groove, providing an inference fit that allows for release of the carrier assembly upon application of minimal force. In addition to the exemplary embodiments described herein, one skilled in the art will appreciate additional means for temporarily securing the carrier assembly within the housing without coupling to the stored energy device 130.

The delivery system 100 may be provided to a practitioner or user fully assembled and/or coated with the agent to be delivered to the skin. In other embodiments, the carrier assembly is provided separately from the housing. In certain preferred embodiments, the stored energy device is provided in the loaded configuration, though it is also possible for the stored energy device to be primed after receipt or shortly before use.

The present disclosure further provides for methods of delivering a microneedle array to a patient's skin surface. One method of delivering a microneedle array using decoupled delivery system 100 is depicted in Figures 5A-5C. Turning initially to Figure 5A, the attachment surface 117 is placed proximate a patient's skin surface 190. Once placed and optionally secured via adhesive layer, a force may be applied to finger engageable portion 122 of the actuator 120. This force is typically applied in a direction 200 that is generally parallel to major plane of array and the attachment surface 117. The applied force moves the wedge 126 relative to the stored energy device, resulting in the application of force orthogonal to the major plane of the stored energy device 130. This application of force causes a transfer of energy (i.e., activation energy) to the stored energy device 130. When the activation energy exceeds a predetermined threshold, the stored energy device releases its potential energy, accelerating a portion of the device 130 towards the carrier assembly 150. In certain embodiments, the force required to release the potential energy is the stored energy device is no greater than 15 N, in some embodiments no greater than 8 N, and in some embodiments no greater than 5 N, and in yet other embodiments no greater than 1 N. In certain circumstances, it may be preferred that the force required be at least 2 N and no greater than 5 N. While it may be advantageous to reduce or minimize activation force, it will be appreciated by those skilled in the art that the activation force should be high enough to avoid inadvertent firing of the stored energy device before the user is ready to use the delivery system.

In releasing its potential energy, at least a portion of the stored energy device 130 travels in the direction of the skin surface. The stored energy device 130 will contact the carrier assembly 150, applying a force in a direction 220 generally orthogonal to the major plane of the microneedle array 152. In certain embodiments, the energy applied by the stored energy device is no greater than 0.3 J, in some embodiments no greater than 0.2 J, and in some embodiments no greater than 0.15 J, and in yet other embodiments no greater than 0.1 J. In certain embodiments, the energy applied by the stored energy device is at least 0.006 J, in some embodiments at least 0.01 J, and in some embodiments at least 0.05 J. In certain circumstances, it may be preferred that the force applied be at least 0.013 J and no greater than 0.12 J. This transfer of the application energy accelerates the carrier assembly 150 including the membrane 140 in the direction of the skin, with the assembly eventually emerging through opening 115. In certain embodiments, the application energy is at least twice the activation energy, in other circumstances at least 5 times the activation energy, in other embodiments at least 10 times the activation energy, in yet other embodiments at least 20 times the activation energy, and in yet other embodiments at least 30 times the activation energy.

Figure 5B depicts the delivery system 100 at a time after the microneedle array 152 has impacted the skin. After transfer of application energy, the motion of the stored energy device 130 slows relative to the motion of the carrier assembly 150 and a measureable gap 230 may form between them. Depending on the physical characteristics of the stored energy device, carrier assembly, and skin, this gap may form and close one or more times before the stored energy device, carrier, and skin come to rest. After impact, the microarray, skin, and membrane continue to move in the direction 220 of the application force. Since the motion is decoupled, however, the carrier assembly 150 is free to move with the skin until the skin's point of maximum extension.

In certain embodiments, the membrane is capable of traveling a greater distance than the carrier assembly would travel due to the energy applied by the stored energy device 130. The length of membrane 140 received in the cavity 116, and the resultant bellowed height 142, can be accordingly designed to allow the membrane 140 to extend substantially beyond the attachment surface 117 of the housing 110.

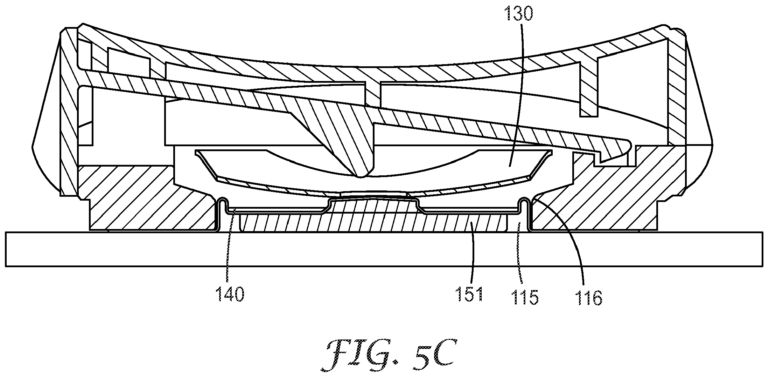

Eventually, the skin and carrier assembly 150 will begin to recoil and dampen. As depicted in Figure 5C, the carrier assembly may come to rest within the cavity 116, with substantially no portion thereof, with the exception of the microneedles, emerging from opening 115. In the depicted embodiment, the membrane 140 does not return to its original bellowed height 142, leaving room in the cavity 116 for the "fired" stored energy device and allowing the microneedles to remain at the desired penetration depth. In other embodiments, at least a portion of the array carrier 151 can emerge from the housing.

Since the user provides a force 200 which is in a direction essentially parallel to the surface of the skin, delivery system 100 can be activated without creating a significant skin dome and without substantially stretching the skin or otherwise disturbing the surface. In certain preferred embodiments, the delivery system does not create a skin dome or stretch the skin. While a small activation force in a direction orthogonal to the skin may be necessary to activate the stored energy device, , the forces generated normal to the skin surface are essentially reacted against one another within the housing 110. Accordingly, the force actually applied to the skin in a direction normal to the plane of the skin is zero or near zero. In addition, the forces generated in a direction parallel to the plane of the skin in this embodiment are at least substantially transferred to the stored energy device as the wedge 126 slides across a surface thereof. Thus, the efficacy of the parallel sliding motion of the actuator will likely discourage the user from pushing the device in a direction normal to the skin during activation, further increasing the consistency of application.

Furthermore, the use of an actuator movable in direction generally parallel to the attachment surface with a high mechanical advantage may allow the activation force of the stored energy device to be set at a higher level, while maintaining a relatively low force required to be applied by the user. This may be particularly useful, as high activation forces can limit the patient population that can operate a particular delivery system. The parallel motion of the actuator may further reduce the variability in velocity of the array at impact.

Figures 8-11 depict additional implementations of a delivery system according to the present disclosure, particularly those featuring alternative actuators. Except as set out in the paragraphs that follow, the delivery systems 800 and 900 are substantially the same as the apparatus 100 described above and consequently a description of the similar aspects need not be repeated.

As depicted in Figures 8 and 9, the delivery system 800 features a rotatable actuator 820 positioned above a stored energy device 830. The rotatable actuator 820 rotates about an axis of rotation 821 that is generally perpendicular to the major plane of the array 852. In other embodiments not depicted herein, the actuator 820 is configured to rotate about an axis angularly offset from axis 821. The rotatable actuator 820 includes helical threads 823 and a graspable ridge 822 on an exterior surface thereof. The upper housing 811 includes an aperture 870 located above the cavity 816 and stored energy device 830. The aperture 870 has one or more side wall portions including helical grooves 873 that correspond to the helical threads on the actuator 820. The rotation of the actuator 820 about the axis 821 brings an engagement surface 824 closer to the stored energy device 830. The actuator 820 may thus be rotated by a user until the engagement surface 824 contacts the stored energy device 830, eventually resulting in application of the predetermined activation force orthogonal to the major plane of the stored energy device 830 without substantial normal force applied to the skin..

Figure 10 and 11 depict a microneedle delivery system 900 featuring a different rotatable actuator. Actuator 920 comprises a cam rotatable about an axis 921. Rotation of the actuator 920 transfers a force orthogonal to the major plane of the stored energy device 930 and/or the microneedle array 952. The delivery systems 800 and 900 may also provide the potential benefits as described above, since equal and opposite forces react within the device leading to no or essentially no force being applied normal to the skin during activation.

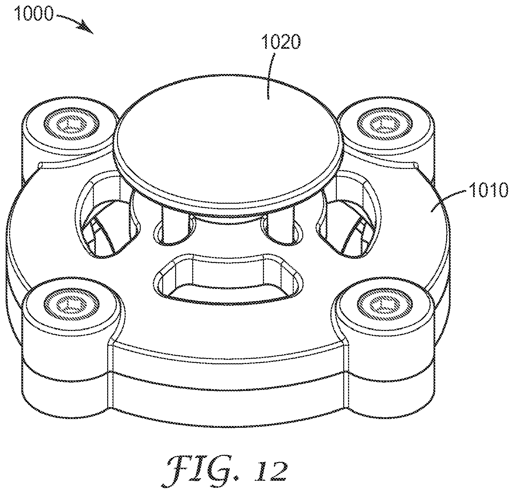

An alternative embodiment of a delivery system is depicted in Figures 12-14. Like the delivery systems discussed above, the delivery system 1000 includes a housing 1010 having a cavity 1016 defined therein; a stored energy device 1030 received in or proximate to the cavity; and a carrier assembly including a microneedle array 1052 coupled to an array carrier 1051. An upper housing portion 1011 includes an attachment cap 1070 received in a central aperture 1019. The attachment cap 1070 can be secured to the upper housing via releasable or other adhesive, interference fit, or other means known to those having skill in the art. Suitable materials for the attachment cap include thermoplastic elastomers, silicones, rubber, and other materials known to one skilled in the art.

The attachment cap 1070 receives the distal end of an elongated arm portion 1053 of the array carrier 1051. In certain embodiments, the end of the elongated arm portion is press fit into aperture 1019 without an attachment cap 1070. The arm portion 1053 also extends through an aperture in the center of stored energy device 1030. In certain embodiments, the arm portion 1053 is designed so that it may be press fit into the attachment cap 1070, while not engaging the periphery of the stored energy device aperture 1032 (i.e., the dimension of the aperture are greater than those of the distal end of the arm portion 1053). In other embodiments, the proximal end of the arm portion 1053 may be configured for an interference fit with the aperture 1032, obviating the need for the attachment cap. In yet other embodiments, the attachment cap and an interference fit between carrier and stored energy device can be used.

An actuator 1020 includes one or more posts 1022 extending from a generally planar base 1021. The posts 1022 extend through apertures 1013 in the upper housing into the cavity 1016 proximate to or in contact with the stored energy device 1030. The base 1021 also includes a center pedestal 1024 designed to engage attachment cap 1070 and/or arm portion 1053. The actuator 1020 may be provided already received housing 1010 or as a separate component.

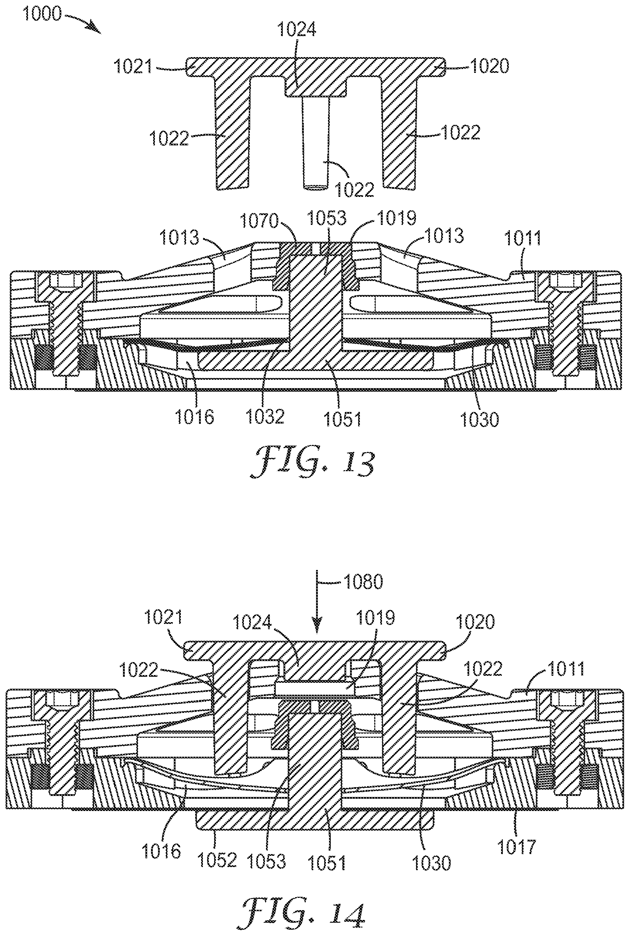

One potential method of using the delivery system 1000 is depicted in Figure 14. A user places actuator 1020 within the apertures in upper housing 1011. A force in a direction 1080 normal to the base 1021 and the attachment surface 1017 is then applied. When force is applied to the base 1021, the center pedestal 1024 pushes the attachment cap 1070 down to a point where it releases from the upper housing 1011 and is free to move independently of the housing 1010. The posts 1022 then engage the stored energy device 1030, transferring the activation energy thereto. The length of the posts 1022 can be designed so that the attachment cap 1070 (or arm portion 1053) is released first, followed by transfer of energy to the stored energy device 1030. In other embodiments, the attachment cap 1070 or arm portion 1053 is released and the stored energy device 1030 is activated at essentially the same time. In yet other embodiments, the application energy transferred to the carrier assembly is sufficient to dislodge the arm portion 1053 from the attachment cap 1070 or the attachment cap 1070 from the housing. Alternatively, portions of the attachment cap 1070 can expand upon application of pressure via the pedestal 1024, releasing the arm portion 1053 while the attachment cap 1070 remains essentially in place.

Once activated, the stored energy device 1030 will contact the carrier assembly 1050, applying a force in a direction 1080 generally orthogonal to the major plane of the microneedle array 1052. This transfer of the application energy accelerates the carrier assembly in the direction of the skin, with the assembly eventually emerging through opening on the bottom of housing 1010. In certain embodiments, the central pedestal 1024 may continue to push the arm portion 1053 towards the skin, potentially resulting in increased penetration depth.

The geometry of the actuator 1020 can be adjusted such that the bottom face of the base 1021 is nearly in contact with the top surface of the upper housing at the time the stored energy device is actuated. In such an embodiment, the total distance that the housing 1010 can rebound away from the skin is limited due to the presence of a user's finger pushing down on the actuator 1020. Alternatively, the actuator 1020 can be designed to provide clearance between the bottom surface of the base 1021 and the top of the upper housing at the time of actuation, which would allow the housing 1010 to move a certain distance away from the skin as the stored energy device 1030 releases its potential energy.

Microneedle arrays suitable for use in the present disclosure may be used to deliver drugs (including any pharmacological agent or agents) through the skin in a variation on transdermal delivery, or to the skin for intradermal or topical treatment, such as vaccination.

In one aspect, drugs that are of a large molecular weight may be delivered transdermally. Increasing molecular weight of a drug typically causes a decrease in unassisted transdermal delivery. Microneedle arrays suitable for use in the present delivery systems have utility for the delivery of large molecules that are ordinarily difficult to deliver by passive transdermal delivery. Examples of such large molecules include proteins, peptides, nucleotide sequences, monoclonal antibodies, DNA vaccines, polysaccharides, such as heparin, and antibiotics, such as ceftriaxone.

In another aspect, microneedle arrays suitable for use in the present invention may have utility for enhancing or allowing transdermal delivery of small molecules that are otherwise difficult or impossible to deliver by passive transdermal delivery. Examples of such molecules include salt forms; ionic molecules, such as bisphosphonates, preferably sodium alendronate or pamedronate; and molecules with physicochemical properties that are not conducive to passive transdermal delivery.

In another aspect, microneedle arrays suitable for use in the present delivery system may have utility for enhancing delivery of molecules to the skin, such as in dermatological treatments, vaccine delivery, or in enhancing immune response of vaccine adjuvants. In one aspect, the drug may be applied to the skin (e.g., in the form of a solution that is swabbed on the skin surface or as a cream that is rubbed into the skin surface) prior to or after applying the microneedle array. In another aspect, the drug or fluid may be applied directly to the microneedles.

In another aspect, the delivery system may be used for creating microprotrusions in the skin.

The delivery systems may be used for immediate delivery, that is where they are applied and immediately removed from the application site, or they may be left in place for an extended time, which may range from a few minutes to as long as 1 week. In one aspect, an extended time of delivery may from 1 to 30 minutes to allow for more complete delivery of a drug than can be obtained upon application and immediate removal. In another aspect, an extended time of delivery may be from 4 hours to 1 week to provide for a sustained release of drug.

Objects and advantages of this invention are further illustrated by the following examples, but the particular materials and amounts thereof recited in these examples, as well as other conditions and details, should not be construed to unduly limit this invention. Unless otherwise indicated, all parts and percentages are by weight.

### Examples

### Microneedle Array Delivery System

A fully assembled delivery system of the design described in Figures 1-5A-C and containing one bifurcating spring was prepared. The housing components of the delivery system were fabricated from ACCURA60 plastic (3D Systems, Rock Hill, SC) using a stereolithographic process. The external housing dimensions were 34.0 mm (diameter) by 9.4 mm (overall height). The opening in the housing for the array was 16.0 mm in diameter.

The bifurcating spring was a four-legged domed spring (Snaptron, Windsor, CO) prepared from 301 full-hard stainless steel. As depicted in Figure 6, the spring had a circular shape with four equally spaced and sized cut-out sections that were shaped as continuous arcs. The diameter (L1) of the spring was 20.2 mm. Each of the four leg regions formed by the cutouts was 4.4 mm as measured along the outer edge of the leg (L2). The distance across and through the center of the spring at the narrowest point was 15.0 mm (L3). A 3.2 mm diameter hole was positioned in the center of the spring. The height of the domed spring before bifurcation and conditioning was 2.3 mm. The stock thickness of the domed spring before forming was 0.20 mm.

Before placing in the assembly, the spring was bifurcated and the activation energy level was set by the following spring conditioning process. The center of the spring was displaced from its resting geometry at a rate of 0.1 mm/second through a bifurcation point until reaching a maximum displacement force of 3000 g after bifurcation. The spring was held at the maximum displacement force for 30 seconds before releasing the applied load. The targeted stored potential energy of the 1-spring system was 0.059 J.

The spring conditioning procedures were designed to provide a spring that produced a total targeted activation force of 350-400 g.

The flexible membrane in the delivery system of was about 25.2 mm in diameter and was constructed from CoTran 9701 polyurethane film (3M Company, St. Paul, MN) having an initial film thickness of 0.05 mm (2 mil.) A film stretching procedure was used to create the bellowed membrane. The membrane was attached to both the lower housing (of an unassembled device) and the array carrier using a continuous coating of 3M Double Coated Medical Tape 1513 (3M Company, St. Paul, MN). The lower housing was simply supported at its perimeter and the membrane film was stretched by displacing the array carrier a distance of 12 mm at a rate of 0.5 mm/second and holding at its maximum displacement for 30 seconds. The assembly of the delivery system was completed by attaching the remaining components and positioning the now bellowed membrane with the attached array carrier in the cavity of the housing.

The microneedle array was molded as an integral portion of the array carrier using either LEXAN HPSIR-1125 polycarbonate (PC) (GE Plastics, Pittsfield, MA) or VECTRA MT 1300 thermoplastic liquid crystal polymer (LCP) (Ticona Engineering Polymers, Florence, KY). The microneedle array featured four-sided pyramidal shaped microneedles having heights of about 500 microns. Each microneedle was formed having a base width of about 167 microns and a tip width of about 10 microns. The microneedles were oriented in an octagon shaped pattern of about 471 microneedles with equal spacing between individual microneedles of about 550 microns (as measured from tip to tip). The array carrier featured a circular shaped base with a diameter of 13.4 mm.

After actuation, the final resting position of the base of the microneedle array extended beyond the base of the lower housing by a distance of 0.11 mm.

### Microneedle Depth of Penetration Study

A study was conducted to determine the depth of penetration (DOP) of the microneedles of an array when applied to the skin surface of Yorkshire cross domestic pigs (Midwest Research Swine, Gibbon, MN), *in vivo.* Prior to application, the microneedle arrays were coated with Rhodamine B using a three-step coating process. In step one, the uncoated arrays were flood coated with a solution containing 50 µl of 1.0 mg/ml polyvinyl alcohol (80% hydrolyzed) (Sigma-Aldrich, St. Louis, MO) and 67 µg/ml of Tween® 80 (Sigma-Aldrich, St. Louis, MO) in 90% (weight/volume) ethyl alcohol. The coated arrays were dried at 35 °C for 20 minutes. In step two, the arrays were flood coated with 60 µl of an aqueous solution of 33.3 mg/ml aluminum potassium sulfate (Penta Manufacturing, Livingston, NJ) and then dried at 35 °C for 30 minutes. In step three, the primed arrays were flood coated with 60 µl of an aqueous solution of 0.08% (weight/volume) Rhodamine B (Sigma-Aldrich, St. Louis, MO) and then dried at 35 °C for 30 minutes.

The ham area of the pig was selected as the application site. The ham area was first trimmed with an electric clipper followed by shaving with a razor and shaving cream. The ham was then rinsed with deionized water and wiped with 70/30 isopropanol water. The animals were anesthetized with isoflurane gas and maintained under anesthesia throughout the experiment.

A fully assembled delivery system as described above was applied to the skin on the ham area of a pig with 3M Double Coated Medical Tape 1513. The delivery system was actuated, maintained on the animal for 15 minutes, and then removed.

The depth of penetration into the pig skin was determined indirectly by measuring the distance from the tip of the microneedle to where the Rhodamine B coating was wiped or dissolved from the microneedle after application into the skin. The measurement was conducted using a Nikon LV-100 microscope at 100X magnification (Nikon Instruments, Melville, NY) with Image Pro® Plus digital image analysis software (Media Cybernetics, Bethesda, MD). For each microneedle array type (PC or LCP), three animals were tested. The mean DOP was determined by sampling a subset of 72 microneedles from each array. Each array pattern was divided into four quadrants and relatively equal numbers of microneedles were sampled from each quadrant. In Table 1, the results are reported from the microneedle DOP study.

**Table 1. Depth of Microneedle Penetration into Pig Skin**

| Composition of Microneedle Array | Mean Microneedle Depth of Penetration (microns) | Standard Deviation (microns) | %RSD |
|---|---|---|---|
| PC | 113 | 14 | 12% |
| LCP | 141 | 42 | 30% |

It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A system (100) for delivering a microneedle array, the system comprising:
a housing (110) having a cavity (116) therein and an attachment surface (117) at least partially surrounding the cavity (116);
a carrier assembly (150) including a solid microneedle array (152) received in the cavity (116);
a stored energy device (130) coupled to a portion of the interior of housing (110) and in contact with a portion of the carrier assembly (150); and
an actuator (120) operable to apply a force to the stored energy device (130) in a direction orthogonal to the major plane of the array (152), the application of the force causing a transfer of activation energy to the stored energy device (130),
wherein the stored energy device (130) comprises a bifurcating spring (330) that undergoes a stepwise motion in a direction orthogonal to a major plane of the spring (330),
wherein the stored energy device (130) is capable of transferring an application energy that is greater than the activation energy,
wherein the stored energy device (130) comprises an aperture (336),
wherein the carrier assembly (150) comprises an elongated arm (1053) received in the aperture (336), and
wherein the elongated arm (1053) is releasably coupled to the housing (110) at a location remote from the array (152).

2. A system for delivering a microneedle array, the system comprising:
a housing (110) having a cavity (116) therein and an attachment surface (117) at least partially surrounding the cavity (116);
a carrier assembly (150) including a solid microneedle array (152) received in the cavity (116);
a stored energy device (130) coupled to a portion of the interior of housing (110) and in contact with a portion of the carrier assembly (150); and
an actuator (120) operable to apply a force to the stored energy device (130) in a direction orthogonal to the major plane of the array (152), the application of the force causing the transfer of an activation energy to the stored energy device (130),
wherein the stored energy device (130) comprises a bifurcating spring (330) that undergoes a stepwise motion in a direction orthogonal to a major plane of the spring (330),
wherein the stored energy device (130) is capable of transferring an application energy that is greater than the activation energy,
wherein the actuator (120) is movable within the housing (110) in a certain direction substantially parallel to the attachment surface (117).

3. The system of claim 1 or claim 2, wherein a ratio of application energy to activation energy is greater than 1.5.

4. The system of claim 3, wherein the ratio is greater than 5.

5. The system of claim 4, wherein the ratio is greater than 20.

6. The system of claims 1-5, wherein the bifurcating spring (330) comprises a domed spring .

7. The system of claims 1-6, wherein the spring (330) comprises a center, and wherein the center of the spring is adapted to travel a certain travel distance upon application of the force, said travel distance in the same direction as the direction of the force transferred to the spring.

8. The system of claim 7, wherein the travel distance is at least 0.5 mm and no greater than 10 mm.

9. The system of claims 1-8 and comprising a plurality of bifurcating springs.

10. The system of any of the preceding claims, wherein the carrier assembly (150) is releasably coupled to the housing (110).

11. The system of claims 1-9, wherein the carrier assembly (150) includes a flexible membrane (140).

12. The system of claim 1, wherein the actuator (120) is movable within the housing (110) in a certain direction orthogonal to the attachment surface.

## Patentansprüche

1. System (100) zur Abgabe einer Mikronadelanordnung, wobei das System aufweist:
ein Gehäuse (110) mit einem darin angeordneten Hohlraum (116) und einer den Hohlraum (116) zumindest teilweise umgebenden Befestigungsfläche (117);
eine Trägerbaugruppe (150), die eine massive Mikronadelanordnung (152) aufweist, die in dem Hohlraum (116) aufgenommen ist;
eine Speicherenergievorrichtung (130), die an einen Abschnitt des Inneren des Gehäuses (110) gekoppelt ist und mit einem Abschnitt der Trägerbaugruppe (150) in Kontakt steht; und
einen Aktuator (120), der betriebsfähig ist, um eine Kraft auf die Speicherenergievorrichtung (130) in einer Richtung orthogonal zur Hauptebene der Anordnung (152) auszuüben, wobei das Ausüben der Kraft eine Übertragung von Aktivierungsenergie auf die Speicherenergievorrichtung (130) bewirkt,
wobei die Speicherenergievorrichtung (130) eine verzweigte Feder (330) aufweist, die eine schrittweise Bewegung in einer Richtung orthogonal zu einer Hauptebene der Feder (330) erfährt,
wobei die Speicherenergievorrichtung (130) in der Lage ist, eine Ausübungsenergie zu übertragen, die größer ist als die Aktivierungsenergie,
wobei die Speicherenergievorrichtung (130) eine Öffnung (336) aufweist,
wobei die Trägerbaugruppe (150) einen langgestreckten Arm (1053) in der Öffnung (336) umfasst, und
wobei der langgestreckte Arm (1053) an einer von der Anordnung (152) entfernten Stelle lösbar an das Gehäuse (110) gekoppelt ist.

2. System zur Abgabe einer Mikronadelanordnung, wobei das System aufweist:
ein Gehäuse (110) mit einem darin angeordneten Hohlraum (116) und einer den Hohlraum (116) zumindest teilweise umgebenden Befestigungsfläche (117);
eine Trägerbaugruppe (150), die eine massive Mikronadelanordnung (152) aufweist, die in dem Hohlraum (116) aufgenommen ist;
eine Speicherenergievorrichtung (130), die an einen Abschnitt des Inneren des Gehäuses (110) gekoppelt ist und mit einem Abschnitt der Trägerbaugruppe (150) in Kontakt steht; und
einen Aktuator (120), der betriebsfähig ist, um eine Kraft auf die Speicherenergievorrichtung (130) in einer Richtung orthogonal zur Hauptebene der Anordnung (152) auszuüben, wobei das Ausüben der Kraft die Übertragung einer Aktivierungsenergie auf die Speicherenergievorrichtung (130) bewirkt,
wobei die Speicherenergievorrichtung (130) eine verzweigte Feder (330) umfasst, die eine schrittweise Bewegung in einer Richtung orthogonal zu einer Hauptebene der Feder (330) erfährt,
wobei die Speicherenergievorrichtung (130) in der Lage ist, eine Ausübungsenergie zu übertragen, die größer ist als die Aktivierungsenergie,
wobei der Aktuator (120) innerhalb des Gehäuses (110) in einer bestimmten Richtung im Wesentlichen parallel zur Befestigungsfläche (117) bewegbar ist.

3. System nach Anspruch 1 oder Anspruch 2, wobei ein Verhältnis von Ausübungs- zu Aktivierungsenergie größer als 1,5 ist.

4. System nach Anspruch 3, wobei das Verhältnis größer als 5 ist.

5. System nach Anspruch 4, wobei das Verhältnis größer als 20 ist.

6. System nach den Ansprüchen 1-5, wobei die verzweigte Feder (330) eine Kuppelfeder aufweist.

7. System nach den Ansprüchen 1-6, wobei die Feder (330) eine Mitte aufweist, und wobei die Mitte der Feder dazu ausgebildet ist, sich bei Ausübung der Kraft über eine bestimmte Bewegungsstrecke zu bewegen, wobei die Bewegungsstrecke in der gleichen Richtung wie die Richtung der Kraft verläuft, die auf die Feder übertragen wird.

8. System nach Anspruch 7, wobei die Bewegungsstrecke mindestens 0,5 mm beträgt und nicht größer als 10 mm ist.

9. System nach den Ansprüchen 1-8, das mehrere verzweigte Federn aufweist.

10. System nach einem der vorstehenden Ansprüche, wobei die Trägerbaugruppe (150) lösbar an das Gehäuse (110) gekoppelt ist.

11. System nach den Ansprüchen 1-9, wobei die Trägerbaugruppe (150) eine flexible Membran (140) aufweist.

12. System nach Anspruch 1, wobei der Aktuator (120) innerhalb des Gehäuses (110) in einer bestimmten Richtung orthogonal zur Befestigungsfläche bewegbar ist.

## Revendications

1. Système (100) pour délivrer une matrice de micro-aiguilles, le système comprenant :
un boîtier (110) ayant une cavité (116) dans celui-ci et une surface de fixation (117) entourant au moins partiellement la cavité (116) ;
un ensemble de support (150) incluant une matrice de micro-aiguilles solide (152) reçue dans la cavité (116) ;
un dispositif d'énergie stockée (130) couplé à une partie de l'intérieur du boîtier (110) et en contact avec une partie de l'ensemble de support (150) ; et
un actionneur (120) opérationnel pour appliquer une force au dispositif d'énergie stockée (130) dans une direction orthogonale par rapport au plan majeur de la matrice (152), l'application de la force provoquant un transfert d'énergie d'activation au dispositif d'énergie stockée (130),
dans lequel le dispositif d'énergie stockée (130) comprend un ressort de bifurcation (330) qui subit un mouvement par paliers dans une direction orthogonale par rapport à un plan majeur du ressort (330),
dans lequel le dispositif d'énergie stockée (130) est capable de transférer une énergie d'application qui est supérieure à l'énergie d'activation,
dans lequel le dispositif d'énergie stockée (130) comprend une ouverture (336),
dans lequel l'ensemble de support (150) comprend un bras allongé (1053) reçu dans l'ouverture (336), et
dans lequel le bras allongé (1053) est couplé de manière amovible au boîtier (110) au niveau d'un emplacement distant de la matrice (152).

2. Système pour délivrer une matrice de micro-aiguilles, le système comprenant :
un boîtier (110) ayant une cavité (116) dans celui-ci et une surface de fixation (117) entourant au moins partiellement la cavité (116) ;
un ensemble de support (150) incluant une matrice de micro-aiguilles solide (152) reçue dans la cavité (116) ;
un dispositif d'énergie stockée (130) couplé à une partie de l'intérieur du boîtier (110) et en contact avec une partie de l'ensemble de support (150) ; et
un actionneur (120) opérationnel pour appliquer une force au dispositif d'énergie stockée (130) dans une direction orthogonale par rapport au plan majeur de la matrice (152), l'application de la force provoquant le transfert d'une énergie d'activation au dispositif d'énergie stockée (130),
dans lequel le dispositif d'énergie stockée (130) comprend un ressort de bifurcation (330) qui subit un mouvement par paliers dans une direction orthogonale par rapport à un plan majeur du ressort (330),
dans lequel le dispositif d'énergie stockée (130) est capable de transférer une énergie d'application qui est supérieure à l'énergie d'activation,
dans lequel l'actionneur (120) est mobile à l'intérieur du boîtier (110) dans une certaine direction sensiblement parallèle à la surface de fixation (117).

3. Système selon la revendication 1 ou la revendication 2, dans lequel un rapport de l'énergie d'application à l'énergie d'activation est supérieur à 1,5.

4. Système selon la revendication 3, dans lequel le rapport est supérieur à 5.

5. Système selon la revendication 4, dans lequel le rapport est supérieur à 20.

6. Système selon les revendications 1 à 5, dans lequel le ressort de bifurcation (330) comprend un ressort en dôme.

7. Système selon les revendications 1 à 6, dans lequel le ressort (330) comprend un centre, et dans lequel le centre du ressort est adapté pour se déplacer d'une certaine distance de déplacement lors de l'application de la force, ladite distance de déplacement dans la même direction que la direction de la force transférée au ressort.

8. Système selon la revendication 7, dans lequel la distance de déplacement est d'au moins 0,5 mm et d'au plus 10 mm.

9. Système selon les revendications 1 à 8 et comprenant une pluralité de ressorts de bifurcation.

10. Système selon l'une quelconque des revendications précédentes, dans lequel l'ensemble de support (150) est couplé de manière amovible au boîtier (110).

11. Système selon les revendications 1 à 9, dans lequel l'ensemble de support (150) inclut une membrane flexible (140).

12. Système selon la revendication 1, dans lequel l'actionneur (120) est mobile à l'intérieur du boîtier (110) dans une certaine direction orthogonale par rapport à la surface de fixation.
